# EUROPEAN PATENT SPECIFICATION

(11) **EP 1 876 994 B1**
(45) Date of publication and mention of the grant of the patent: **26.07.2017**
(21) Application number: 06751748.2
(22) Date of filing: 27.04.2006
(51) Int. Cl.: A61C 13/087, A61K 6/083

(54) **MALLEABLE SYMMETRIC DENTAL CROWNS**
FORMBARE SYMMETRISCHE ZAHNKRONEN
COURONNES DENTAIRES SYMETRIQUES MALLEABLES

(30) Priority: 29.04.2005 US 676433 P
(43) Date of publication of application: 16.01.2008
(73) Proprietor: 3M Innovative Properties Company, St. Paul, MN 55133-3427 (US)
(72) Inventor: KARIM, Naimul, Saint Paul, MN 55133-3427 (US); BIEGLER, Robert M.,, Saint Paul, MN 55133-3427 (US); COLBURN, David J.,, Saint Paul, MN 55133-3427 (US); JACOBS, Dwight W.,, Saint Paul, MN 55133-3427 (US); KECK, Steven C.,, Falcon Heights, MN 55113 (US)
(74) Representative: Vossius & Partner Patentanwälte Rechtsanwälte mbB
(86) International application number: PCT/US2006/016197
(87) International publication number: WO 2006/119003

(56) References cited:
- WO-A-2005/018484
- GB-A- 1 051 735
- US-A- 4 778 386
- US-A1- 2003 039 943
- US-A1- 2003 114 553

## Description

The present invention relates to the field of preformed dental crowns used in restorative dentistry and methods of using the preformed dental crowns.

Restorative dentistry is an important market in today's dental industry. In particular, tooth repair with temporary and permanent crowns is a common procedure, typically requiring multiple dental appointments. In many instances, practitioners rely on preformed dental crowns to expedite the restoration process by providing a dental crown in the shape of the tooth being restored.

Preformed crowns that are available in the market today are typically made of metals (e.g., stainless steel, aluminum, metal alloys, etc.) or polymers (e.g. polycarbonate, polyacetal, etc.). Metal crowns can additionally be covered with a tooth colored coating to provide an aesthetic appearance.

If adjustments to the preformed metal and polymer crowns are needed, they can be trimmed either with a crown scissors, heatless stone, or other instruments to remove material at the crown margin to obtain a desired crown length. Metal crowns may also be crimped at the cervical region to obtain good marginal adaptation. Modification of other crown dimensions, however, such as interproximal distances, crown anatomy, etc. are not performed because the materials used in the preformed crowns are not amenable to shape adjustment by the practitioner.

Because the shape of conventional preformed crowns cannot be adjusted, the preformed crowns are offered in many different shapes to cover different tooth forms (as well as in different sizes). As a result, the total number of preformed crown sizes and shapes offered in conventional preformed dental crowns is quite large. In part, the large number of preformed crowns is driven by the need to provide different preformed crowns for the left and right arches.

WO 2005/018484 A relates to methods of manufacturing hardenable dental articles, packaged hardenable dental articles, and methods of packaging hardenable dental articles. The manufacturing may involve molding a hardenable dental material in a mold cavity that may be lined with a mold liner. The mold body may also form the package of the hardenable dental article formed within the mold cavity. The hardenable dental articles may be provided in mold cavities located in sacrificial mold bodies that may be torn, stretched, softened, dissolved, etc. to release the hardenable dental articles in the mold cavities.

### SUMMARY OF THE INVENTION

The present invention provides preformed dental crowns that have side-generic external shapes with a plane of symmetry in the facial-lingual direction. The preformed dental crowns also include a hardenable composition that has sufficient malleability such that the side-generic external shape can be formed into, e.g., a left-side specific or right-side specific adjusted external shape.

The present invention may include preformed dental crowns in different external shapes corresponding to a variety of tooth anatomies (e.g., incisors, canines, premolars, and molars). In addition to being side-generic as discussed herein, at least some of the preformed dental crowns may also be used on the upper (maxillary) arch or lower (mandibular) arch with or without modifications to adapt them for use on those arches. For example, smaller incisors may potentially be used as mandibular incisors in some patients and maxillary incisors in others. Similarly, premolars and molars may potentially be used interchangeably on the maxillary or mandibular arches if the malleability of the hardenable composition of the preformed dental crown is sufficient.

Another potential advantage of providing preformed dental crowns with side-generic molar shapes (having a plane of symmetry in the facial-lingual direction) is that the preformed dental crowns may be used as first, second, or third molars or first or second premolars (with some adjustments/forming as required). This may further reduce the inventory requirements for practitioners as discussed herein.

In addition, it may be advantageous to provide some of the preformed dental crowns intended for use as premolars or molars with a plane of symmetry in the mesial-distal direction and the facial-lingual direction. The second plane of symmetry may make such preformed dental crowns more easily adapted to use in multiple locations (e.g., on the upper or lower arch, in different molar locations, etc.).

In some instances, however, it may be advantageous to provide preformed dental crowns of the present invention in side-generic external shapes that are more specifically directed to use on only one of the maxillary or mandibular arches (as opposed to external shapes that are more generic to use on either the maxillary or mandibular arches).

The symmetric preformed dental crowns of the present invention may preferably provide manufacturers and practitioners with the opportunity to significantly reduce the inventory of preformed dental crowns. By providing symmetric preformed dental crowns that can (with some potential shape adjustments) be used in left-side or right-side locations the number of preformed dental crowns required may be cut in half. Further inventory reductions may be possible if some of the preformed dental crowns may be used in multiple locations (e.g., the first, second, and third molar or first or second premolars). Even more inventory reductions may be possible if at least some of the preformed dental crowns may be used on the upper or lower arch as needed.

As used herein to describe the external shape of preformed dental crowns, "side-generic" (and variations thereof) means that the external shapes of the preformed dental crowns do not contain all of the left-side or right-side specific anatomical features that would be associated with an anatomically correct preformed dental crown. Preferably, the preformed dental crowns with side-generic external shapes of the present invention would require at least some forming or adjustment into a left-side specific or right-side specific adjusted external shape when used.

As used herein, "symmetry" (and variations thereof) is used in a functional sense in that the side-generic external shapes of the preformed dental crowns are symmetrical about a plane or planes that allow crowns to be used on the right or left arch as needed. Because, however, the preformed dental crowns are side-generic, i.e., they are not specific to the left or right side, some customization by the practitioner is typically required for proper fit. As used herein, the term "symmetry" (and variations thereof) is not used to mean perfect geometric symmetry about a plane or planes, i.e., some deviation from perfect geometric symmetry is allowable.

The term "self-supporting" as used herein means that each crown is dimensionally stable and will maintain its preformed shape without significant deformation at room temperature (i.e., about 20°C to about 25°C) for at least about two weeks when freestanding (i.e., without the support of packaging or a container). Preferably, the preformed dental crowns of the present invention are dimensionally stable at room temperature for at least about one month, and more preferably, for at least about six months. Preferably, the preformed dental crowns of the present invention are dimensionally stable at temperatures above room temperature, more preferably up to about 40°C, even more preferably up to about 50°C, and even more preferably up to about 60°C. This definition applies in the absence of conditions that activate any initiator system and in the absence of an external force other than gravity.

The term "sufficient malleability" means that the self-supporting preformed dental crown is capable of being custom-shaped and fitted into a left-side specific or right-side specific adjusted external shape, for example, to a patient's mouth, under a moderate manual force (i.e., a force that ranges from light finger pressure to that applied with manual operation of a small hand tool, such as a dental composite instrument). The shaping, fitting, forming, etc. are performed by adjusting the external shape of the preformed dental crown without removing or adding material to the preformed dental crown in areas other than the margin (which may be trimmed if required).

In some instances, it may be preferred that the preformed dental crowns of the present invention consist essentially of a hardenable composition.

The hardenable compositions used in preformed dental crowns of the present invention may exhibit the desired "sufficient malleability" at temperatures of, e.g., 40 degrees Celsius or less. In still other instances, the hardenable compositions may exhibit "sufficient malleability" in a temperature range of, e.g., 15°C to 38°C.

It may be further preferred that the hardenable compositions of the preformed dental crowns of the present invention be "irreversibly hardenable" which, as used herein, means that after hardening such that the composition loses its malleability it cannot be converted back into malleable form without destroying the external shape of the dental crown.

Examples of some potentially suitable hardenable compositions that may be used to construct the preformed dental crowns of the invention with sufficient malleability may include, e.g., hardenable organic compositions (filled or unfilled), polymerizable dental waxes, hardenable dental compositions having a wax-like or clay-like consistency in the unhardened state, etc. It may be preferred that, in some embodiments, the preformed dental crowns of the present invention be constructed of hardenable compositions that consist essentially of non-metallic materials.

Potentially suitable hardenable compositions that may be used to manufacture the preformed dental crowns of the present invention may be described in U.S. Patent Application Publication No. US 2003/0114553, titled HARDENABLE SELF-SUPPORTING STRUCTURES AND METHODS (Karim et al.). Other suitable hardenable compositions may include those described in U.S. Patent Nos. 5,403,188 (Oxman et al.); 6,057,383 (Volkel et al.); and 6,799,969 (Sun et al.).

With respect to the hardenable compositions described in US 2003/0114553, the unique combination of highly malleable properties (preferably without heating above room temperature or body temperature) before hardening (e.g., cure) and high strength (preferably, e.g., a flexural strength of at least about 25 MPa) after hardening may provide preformed dental crowns with numerous potential advantages.

In one aspect, the present invention provides a dental article that includes a preformed dental crown with a self-supporting side-generic external shape selected from the group consisting of incisor, canine, premolar, and molar; wherein the side-generic external shape has a plane of symmetry in facial-lingual direction; and wherein the preformed dental crown includes a hardenable composition that has sufficient malleability such that the side-generic external shape can be formed into a left-side specific or right-side specific adjusted external shape. In some embodiments, the side-generic external shape may be further selected from the group consisting of premolar and molar; wherein the side-generic external shape also includes a plane of symmetry in the mesial-distal direction.

In another aspect, the present invention provides a dental article that includes a preformed dental crown with a self-supporting side-generic external shape selected from the group consisting of premolar and molar; wherein the side-generic external shape has a plane of symmetry in both the facial-lingual direction and the mesial-distal direction; and wherein the preformed dental crown includes a hardenable composition that has sufficient malleability such that the side-generic external shape can be formed into a left-side specific or right-side specific adjusted external shape.

In another aspect, the present invention provides a set of dental articles, the set including one or more preformed dental crowns with a self-supporting side-generic external shape of an incisor, wherein the side-generic external shape has a plane of symmetry in facial-lingual direction; one or more preformed dental crowns with a self-supporting side-generic external shape of a canine, wherein the side-generic external shape has a plane of symmetry in facial-lingual direction; one or more preformed dental crowns with a self-supporting side-generic external shape of a premolar, wherein the side-generic external shape has a plane of symmetry in the facial-lingual direction; one or more preformed dental crowns with a self-supporting side-generic external shape of a molar, wherein the side-generic external shape has a plane of symmetry in the facial-lingual direction; and a package containing the set of dental articles; wherein each preformed dental crown in the set of preformed dental crowns includes a hardenable composition that has sufficient malleability such that the side-generic external shape can be formed into a left-side specific or right-side specific adjusted external shape. In some embodiments, the one or more preformed dental crowns with a self-supporting side-generic external shape of a premolar have also a plane of symmetry in the mesial-distal direction. In some embodiments, the one or more preformed dental crowns with a self-supporting side-generic external shape of a molar have also a plane of symmetry in the mesial-distal direction.

In another aspect, the present invention may provide a dental article that includes a preformed dental crown with a self-supporting side-generic external shape selected from the group consisting of premolar and molar; wherein the side-generic external shape has also a plane of symmetry in mesial-distal direction; and wherein the preformed dental crown includes a hardenable composition that has sufficient malleability such that the side-generic external shape can be formed into a left-side specific or right-side specific adjusted external shape.

The methods of using the preformed dental crowns of the present invention include forming the hardenable compositions in the crowns to include, e.g., left-side specific features or right-side specific features; hardening the hardenable compositions by, e.g., exposing them to actinic radiation.

These and other features and advantages of the present invention may be described below in connection various exemplary embodiments of the present invention.

### BRIEF DESCRIPTIONS OF THE FIGURES

FIG. 1 is an occlusal view of a preformed dental crown for a mandibular molar with a facial-lingual plane depicted on edge in the figure.
FIG. 2 is a facial side view of the preformed dental crown of FIG. 1 with a facial-lingual plane depicted on edge in the figure.
FIG. 3 is an incisal-edge view of a preformed dental crown for a maxillary incisor with a facial-lingual plane depicted on edge in the figure.
FIG. 4 is a facial side view of the preformed dental crown of FIG. 3 with a facial-lingual plane depicted on edge in the figure.
FIG. 5 is an occlusal view of a preformed dental crown for a mandibular molar with a facial-lingual plane and a mesial-distal plane depicted on edge in the figure.
FIG. 6 is a mesial side view of the preformed dental crown of FIG. 5 with a mesial-distal plane depicted on edge in the figure.
FIG. 7 is an occlusal view of a conventional asymmetric preformed dental crown shaped to replace a mandibular first molar.
FIG. 8 is a facial side view of the conventional asymmetric preformed dental crown of FIG. 7.
FIG. 9 is an incisal-edge view of a conventional asymmetric preformed dental crown shaped to replace a right maxillary central incisor.
FIG. 10 is a facial side view of the conventional asymmetric preformed dental crown of FIG. 9.
FIG. 11 is a cross-sectional view of a preformed dental crown of the present invention taken along the facial-lingual plane to illustrate an offset cavity within the dental crown.

### DESCRIPTION OF EXEMPLARY EMBODIMENTS OF THE INVENTION

In the following detailed description of exemplary embodiments of the invention, reference is made to the accompanying figures of the drawing which form a part hereof, and in which are shown, by way of illustration, specific embodiments in which the invention may be practiced. It is to be understood that other embodiments may be utilized and structural changes may be made, the scope of the present invention being defined by the appended claims.

The present invention provides preformed dental crowns that have side-generic external shapes with a plane of symmetry in the facial-lingual direction. The preformed dental crowns also include a hardenable composition that has sufficient malleability such that the side-generic external shape can be formed into, e.g., a left-side specific or right-side specific adjusted external shape.

As discussed herein, the hardenable compositions of the preformed dental crowns with their side-generic external shapes may preferably be formed or adjusted for use in either left-side or right-side locations within the mouth. Because the dental crowns are formed with side-generic external shapes, use of the preformed dental crowns may preferably involve some forming of the hardenable composition by a practitioner during fitting to form or adjust the side-generic external shapes of the preformed dental crowns to include left-side specific or right-side specific features. The end result may preferably be a dental crown with a left-side specific or right-side specific adjusted external shape. Hardening of the hardenable composition of the dental crown can then preferably fix the adjusted external shape for use in the mouth.

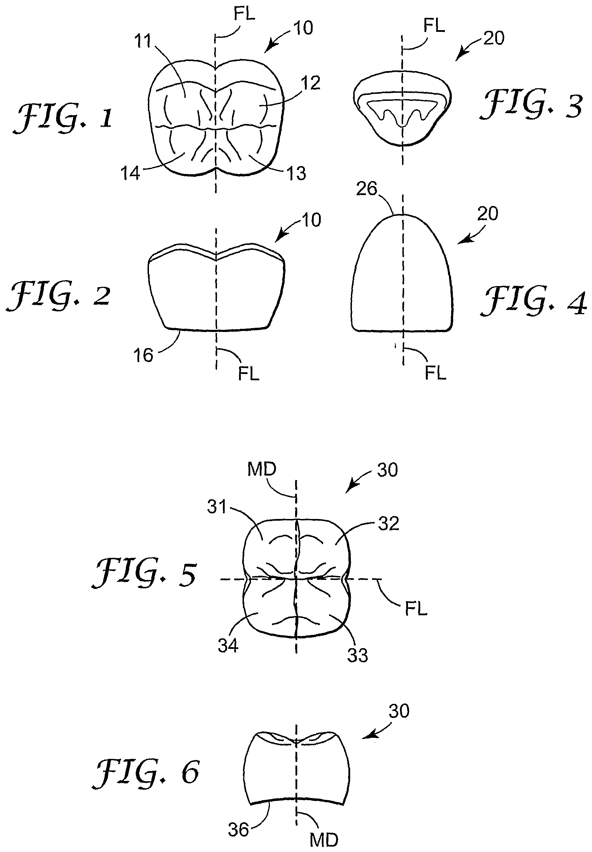

One exemplary symmetric preformed dental crown 10 with a side-generic external shape according to the present invention is depicted in FIGS. 1 and 2. FIG. 1 is an occlusal view of the preformed dental crown 10 for a mandibular molar having symmetry about a facial-lingual plane FL (which is depicted as a broken line in FIG. 1). FIG. 2 is a facial side view of the preformed dental crown 10 depicting symmetry about the facial-lingual plane FL (which is depicted as a broken line).

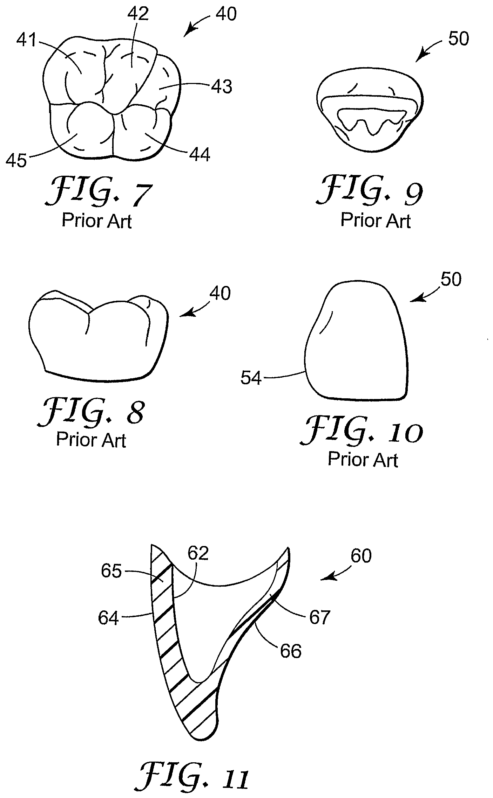

As seen in FIGS. 1 and 2, the side-generic external shape of the crown 10 has symmetry about the facial-lingual plane FL in both the occlusal and facial side views. That symmetry can be contrasted with the asymmetric external shape of a prior art crown 40 designed to replace a side-specific mandibular first molar as depicted in FIGS. 7 & 8. The side-specific external shape of the prior art crown 40 does not exhibit symmetry about any plane.

In addition to its lack of symmetry, the prior art crown 40 includes five occlusal cusps 41, 42, 43, 44 & 45 (as seen in FIG. 7). That is in contrast to the four occlusal cusps 11, 12, 13 & 14 on the dental crown 10 of the present invention. Hardenable preformed dental crowns according to the present invention may preferably exhibit such anatomic generalizations or simplifications to provide the desired symmetry.

It may be preferred for example, that at least some of the symmetric preformed dental crowns of the present invention adapted for replacement of maxillary and/or mandibular premolars or molars may include either fewer or more anatomical features than an anatomically correct dental crown.

Use of the symmetric preformed dental crown 10 to replace, e.g., a mandibular first molar, may preferably involve adjustment or forming of the symmetric preformed dental crown 10 to more closely approximate the left-side or right-side specific anatomy of the tooth being replaced. Such adjustments are possible using compositions that have sufficient malleability as discussed herein. The adjusted dental crown may preferably be hardened after the forming such that it retains the adjusted external shape in use in the mouth.

The preformed dental crown 10 includes a margin 16 at the base of the crown 10. As discussed herein, although forming of the hardenable material of the preformed dental crown 10 to provide an adjusted external shape preferably involves manipulation, but not removal or addition of material to the crown 10, it is envisioned that a practitioner may remove material from the margin 16 to improve the fit of the crown in a selected location.

To generate a preformed dental crown of the present invention that is generic to the right and left sides of the mouth, some generalization of the anatomies may be required. In one approach, elements of natural tooth anatomy may be selected that will work best in multiple locations within the mouth. In some cases, that may involve selecting the half of the tooth anatomy that will work best for mirroring and then mirroring that half about the plane of symmetry. In other cases, a designer may select the half of the tooth anatomy that is best in terms of functionality, aesthetics, etc. and then mirror it about a plane of symmetry. In the case of occlusal cusps for some molars, it may be advantageous to remove some anatomical features that, if mirrored, would lead to an unacceptable crown in terms of appearance and/or functionality. That simplified shape could then be mirrored about the plane of symmetry. In another approach, anatomical features from multiple tooth anatomies could be combined, e.g., first and second molars, to create a hybrid dental crown half followed by mirroring the hybrid dental crown half about the plane of symmetry.

Although the above techniques may be useful for developing symmetric preformed dental crowns to replace more complex anatomical tooth shapes such as those found posteriorly within the mouth, the development of symmetric preformed dental crowns according to the present invention for the incisors and canines may be more straightforward in the absence of the more complex occlusal cusps found on the posterior teeth.

FIGS. 3 & 4 are views of a symmetric preformed dental crown 20 with a side-generic external shape that is designed to replace an incisor (preferably a maxillary incisor). FIG. 3 is an incisal-edge view of a preformed dental crown 20 having symmetry about a facial-lingual plane FL (the plane being depicted on edge by a broken line in both FIGS. 3 & 4). FIG. 4 is a facial side view of the preformed dental crown 20 depicting its symmetry about a facial-lingual plane FL.

The symmetry of the preformed dental crown 20 of FIGS. 3 & 4 can be contrasted with the asymmetric external shape of a prior art crown 50 designed to replace a right maxillary central incisor as depicted in FIGS. 9 & 10. The external shape of the prior art location-specific crown 50 does not exhibit symmetry about a facial-lingual plane FL as seen in FIG. 10.

Use of the symmetric preformed dental crown 20 to replace, e.g., a right maxillary central incisor, may preferably involve adjustment or forming of the symmetric preformed dental crown 20 to more closely approximate the right-side specific anatomy of the tooth being replaced (e.g., forming to add the bulge 54 along the distal side of the tooth). Such adjustments are possible using hardenable compositions that have sufficient malleability as discussed herein. The hardenable composition of the adjusted dental crown 20 may preferably be hardened after the forming such that crown 20 retains the side-specific adjusted external shape in use in the mouth.

The preformed dental crown 20 includes a margin 26 at the base of the crown 20. As discussed herein, although forming of the hardenable material of the preformed dental crown 20 to provide an adjusted external shape preferably involves manipulation, but not removal or addition of material to the crown 20, it is envisioned that a practitioner may remove material from the margin 26 to improve the fit of the crown in a selected location.

FIGS. 5 & 6 depict a symmetric preformed dental crown 30 of the present invention that is designed to replace a molar. FIG. 5 is an occlusal view of a preformed dental crown 30 in which its symmetry about both a facial-lingual plane FL and mesial-distal plane MD can be seen (the planes being depicted on edge by broken lines). The dental crown 30 includes occlusal cusps 31, 32, 33, and 34. FIG. 6 is a mesial side view of the preformed dental crown 30 in which its symmetry about the mesial-distal plane MD can be seen. It should be noted that the preformed dental crown 30 does not exhibit perfect geometric symmetry about the facial-lingual plane FL or the mesial-distal plane MD and is, thus, exemplary of the concept that preformed dental crowns of the present invention preferably exhibit symmetry in the functional sense as discussed herein.

Use of the symmetric preformed dental crown 30 to replace, e.g., a mandibular molar, may preferably involve adjustment or forming of the symmetric preformed dental crown 30 to more closely approximate the left-side or right-side specific anatomy of the tooth being replaced. In addition, adjustments may be made to improve the fit of the crown for different molar locations (e.g., first, second, or third molar locations or even first or second premolar locations). Such adjustments are possible using hardenable compositions that have sufficient malleability as discussed herein. The hardenable composition of the adjusted dental crown 30 may preferably be hardened after the forming such that dental crown 30 retains the side-specific adjusted external shape after hardening.

The preformed dental crown 30 includes a margin 36 at the base of the crown 30. As discussed herein, although forming of the hardenable material of the preformed dental crown 30 to provide an adjusted external shape preferably involves manipulation, but not removal or addition of material to the crown 30, it is envisioned that a practitioner may remove material from the margin 36 to improve the fit of the crown in a selected location.

FIG. 11 depicts another optional feature that may be provided in the preformed dental crowns of the present invention. In the cross-sectional view of FIG. 11, the depicted preformed dental crown 60 includes a cavity 62 formed within the interior of the dental crown 60. The cross-sectional view is taken such that FIG. 11 includes facial surface 64 located on one side of the cavity 62 and the lingual surface 66 located on the opposite side of the cavity 62.

Although the external shape of the crown 60 may preferably exhibit symmetry as described herein, that symmetry may not necessarily be present in the interior features of the preformed dental crowns of the present invention. In the crown 60 of FIG. 11, for example, the cavity 62 may preferably be offset towards the lingual direction, i.e., the lingual wall 67 of the crown 60 as formed between the cavity 62 and the lingual surface 66 may be thinner than the facial wall 65 formed between the cavity 62 and the facial surface 64.

An offset cavity may offer some potential advantages in that it may simplify adaptation of the crown to the prepared tooth by allowing an easier and more anatomical contouring on the lingual side. For example, full crown preparation of incisors often involves more tooth reduction on the facial side than on the lingual side of the tooth. As a result, a crown with a corresponding offset cavity may be more easily fitted onto the prepared tooth. Another potential advantage of an offset cavity in the dental crowns of the present invention may be that the thicker facial wall 65 could provide improved hiding power for temporary cements (which are often opaque). Reduced visibility of an opaque cement layer on the prepared tooth can improve the aesthetic appearance of the dental crowns, particularly for those in more visible locations.

The preformed dental crowns of the present invention may be manufactured by any suitable technique. Examples of some potentially suitable techniques for manufacturing self-supporting preformed dental crowns may be described in, e.g., International Publication No. WO 2005/018484 A2, titled HARDENABLE DENTAL ARTICLE AND METHOD OF MANUFACTURING THE SAME (Karim et al.).

### HARDENABLE COMPOSITIONS

As discussed herein, the preformed dental crowns with side-generic external shapes preferably include hardenable compositions that are sufficiently malleable to facilitate forming of preformed dental crowns with side-generic shapes into left-side specific or right-side specific adjusted external shapes during the fitting process. Because the compositions are hardenable, the adjusted external shape can preferably be retained.

Potentially useful hardenable compositions for the preformed dental crowns of the present invention may include, e.g., thermosetting polymers, polymerizable waxes, hardenable organic materials (filled or unfilled), etc. Some potentially suitable hardenable compositions may include those described in U.S. Patent Nos. 5,403,188 (Oxman et al.); 6,057,383 (Volkel et al.); and 6,799,969 (Sun et al.).

Other potentially preferred hardenable compositions that may be used to manufacture the preformed dental crowns of the present invention may be described in U.S. Patent Application Publication No. US 2003/0114553, titled HARDENABLE SELF-SUPPORTING STRUCTURES AND METHODS (Karim et al.). As described therein (and briefly summarized in the following discussion), a hardenable composition of US 2003/0114553 may include a resin system that includes a crystalline component, greater than 60 percent by weight (wt-%) of a filler system (preferably, greater than 70 wt-% of a filler system), and an initiator system, wherein the hardenable composition exhibits sufficient malleability to be formed into a side-specific adjusted external shape, preferably at a temperature of about 15°C to 38°C (more preferably, about 20°C to 38°C, which encompasses typical room temperatures and body temperatures, and most preferably, at room temperature). It may be preferred that the compositions of the present invention do not need to be heated above body temperature (or preferably, even about room temperature) to become malleable as discussed herein.

Typically and preferably, at least a portion of the filler system of the hardenable compositions of US 2003/0114553 includes particulate filler. Preferably, in this and various other embodiments, if the filler system includes fibers, the fibers are present in an amount of less than 20 wt-%, based on the total weight of the composition.

The crystalline component may preferably provide a morphology that assists in maintaining the self-supporting first shape (i.e., side-generic external shape). This morphology includes a noncovalent structure, which may be a three-dimensional network (continuous or discontinuous) structure. If desired, the crystalline component can include one or more reactive groups to provide sites for polymerizing and/or crosslinking. If such crystalline components are not present or do not include reactive groups, such reactive sites are provided by another resin component, such as an ethylenically unsaturated component.

Thus, for certain embodiments, the resin system preferably includes at least one ethylenically unsaturated component. Preferred ethylenically unsaturated components are selected from the group consisting of mono-, di-, or poly-acrylates and methacrylates, unsaturated amides, vinyl compounds (including vinyl oxy compounds), and combinations thereof. This ethylenically unsaturated component can be the crystalline component, although in certain preferred embodiments it is noncrystalline.

The crystalline component can include polyesters, polyethers, polyolefins, polythioethers, polyarylalkylenes, polysilanes, polyamides, polyurethanes, or combinations thereof. Preferably, the crystalline component includes saturated, linear, aliphatic polyester polyols containing primary hydroxyl end groups. The crystalline component can optionally have a dendritic, hyperbranched, or star-shaped structure, for example.

The crystalline component can optionally be a polymeric material (i.e., a material having two or more repeat units, thereby including oligomeric materials) having crystallizable pendant moieties and the following general formula: 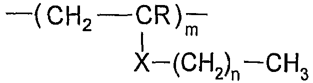 wherein R is hydrogen or a (C₁-C₄)alkyl group, X is --CH₂--, --C(O)O--, --O-C(O)--, --C(O)-NH--, --HN-C(O)--, --O--, --NH--, -O-C(O)-NH-, -HN-C(O)-O-,-HN-C(O)-NH--, or --Si(CH₃)₂--, m is the number of repeating units in the polymer (preferably, 2 or more), and n is great enough to provide sufficient side chain length and conformation to form polymers containing crystalline domains or regions.

Alternative to, or in combination with, the crystalline component, the hardenable composition can include a filler that is capable of providing a morphology to the composition that includes a noncovalent structure, which may be a three-dimensional network (continuous or discontinuous) structure, that assists in the maintenance of the first shape. Preferably, such a filler has nanoscopic particles, more preferably, the filler is an inorganic material having nanoscopic particles. To enhance the formation of the noncovalent structure, the inorganic material can include surface hydroxyl groups. Most preferably, the inorganic material includes fumed silica.

Furthermore, the use of one or more surfactants can also enhance the formation of such a noncovalent structure. A potentially preferred composition may include, in addition to a resin system and an initiator system, either a crystalline component, or a filler system that includes a nanoscopic particulate filler (preferably, both a micron-size particulate filler and a nanoscopic particulate filler) and a surfactant system, or both a crystalline component and a filler system and surfactant system. As used herein, a filler system includes one or more fillers and a surfactant system includes one or more surfactants.

Another potential embodiment of the hardenable compositions that may be used in the preformed dental crowns of the invention may include a hardenable composition of US 2003/0114553 that includes a resin system, a filler system at least a portion of which is an inorganic material having nanoscopic particles with an average primary particle size of no greater than about 50 nanometers (nm), a surfactant system, and an initiator system. The hardenable composition may preferably exhibit sufficient malleability to be formed into a side-specific adjusted external shape at a temperature of about 15°C to 38°C. In embodiments with a surfactant system and nanoscopic particles, the resin system may preferably include at least one ethylenically unsaturated component, and the filler system is present in an amount of greater than 50 wt-%.

In other potentially preferred embodiments, hardenable compositions of the present invention may include a resin system that includes: a noncrystalline component selected from the group consisting of mono-, di-, or poly- acrylates and methacrylates, unsaturated amides, vinyl compounds, and combinations thereof; and a crystalline component selected from the group consisting of polyesters, polyethers, polyolefins, polythioethers, polyarylalkylenes, polysilanes, polyamides, polyurethanes, polymeric materials (including oligomeric materials) having crystallizable pendant moieties and the following general formula: 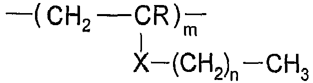 wherein R is hydrogen or a (C₁-C₄)alkyl group, X is --CH₂--, --C(O)O--, --O-C(O)--,-C(O)-NH--, --HN-C(O)--, --O--, --NH--, or -O-C(O)-NH-, -HN-C(O)-O-, --HN-C(O)-NH--, or --Si(CH₃)₂--, m is the number of repeating units in the polymer (preferably, 2 or more), and n is great enough to provide sufficient side chain length and conformation to form polymers containing crystalline domains or regions, and combinations thereof. The hardenable composition further includes greater than about 60 wt-% of a filler system and an initiator system. The hardenable composition preferably exhibits sufficient malleability to be formed into a side-specific adjusted external shape at a temperature of about 15°C to 38°C. If the filler system includes fibers, the fibers may preferably be present in an amount of less than 20 wt-%, based on the total weight of the hardenable composition.

In yet another potentially preferred embodiment, hardenable compositions of the present invention may include a resin system with a crystalline compound of the formula: 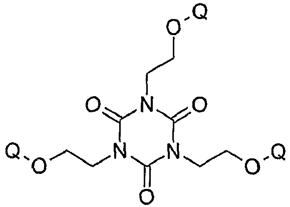 wherein each Q independently comprises polyester segments, polyamide segments, polyurethane segments, polyether segments, or combinations thereof; a filler system; and an initiator system.

As used herein and in the appended claims, the singular forms "a," "and," and "the" include plural referents unless the context clearly dictates otherwise. Thus, for example, reference to "a" or "the" component may include one or more of the components and equivalents thereof known to those skilled in the art.

Exemplary embodiments of this invention are discussed and reference has been made to some possible variations within the scope of this invention. These and other variations and modifications in the invention will be apparent to those skilled in the art without departing from the scope of the invention, and it should be understood that this invention is not limited to the exemplary embodiments set forth herein. Accordingly, the invention is to be limited only by the claims provided below.

## Claims

1. A dental article comprising:
a preformed dental crown (10; 20; 30) that comprises a self-supporting side-generic external shape selected from the group consisting of incisor, canine, premolar, and molar;
wherein the side-generic external shape is symmetrical about a plane of symmetry in facial-lingual direction (FL);
and wherein the preformed dental crown (10; 20; 30) comprises a hardenable composition that has sufficient malleability such that the side-generic external shape can be formed into a left-side specific or right-side specific adjusted external shape.

2. A dental article according to claim 1, wherein the side-generic external shape is further selected from the group consisting of premolar and molar; and wherein the side-generic external shape comprises a plane of symmetry in the mesial-distal direction (MD).

3. A dental article according to claim 1, wherein the preformed dental crown (10; 20; 30; 60) comprises an internal cavity (62), and wherein the internal cavity (62) is offset towards the lingual direction or the facial direction.

4. A dental article according to claim 1, wherein the hardenable composition has sufficient malleability to be formed into the left-side specific or right-side specific adjusted external shape at a temperature of 15°C to 38°C.

5. A dental article according to claim 1, wherein the hardenable composition comprises a resin system comprising a crystalline component, greater than 60 wt-% of a filler system, and an initiator system, wherein the hardenable composition exhibits the sufficient malleability at a temperature of about 15°C to 38°C, with the proviso that if the filler system comprises fibers, the fibers are present in an amount of less than 20 wt-%, based on the total weight of the hardenable composition.

6. A dental article according to claim 1, wherein the hardenable composition comprises a resin system comprising a crystalline component, greater than 70 wt-% of a filler system, and an initiator system, with the proviso that if the filler system comprises fibers, the fibers are present in an amount of less than 20 wt-%, based on the total weight of the composition.

7. A dental article according to claim 1, wherein the hardenable composition comprises:
a resin system comprising at least one ethylenically unsaturated component and a crystalline component;
greater than 60 wt-% of a filler system; and
an initiator system;
wherein the hardenable composition exhibits the sufficient malleability at a temperature of about 15°C to 38°C.

8. A dental article according to claim 1, wherein the hardenable composition comprises:
a resin system comprising at least one ethylenically unsaturated component;
greater than 50 wt-% of a filler system at least a portion of which is an inorganic material comprising nanoscopic particles having an average primary particle size of no greater than about 50 nm;
an initiator system; and
a surfactant system.

9. A dental article according to claim 1, wherein the hardenable composition comprises:
a resin system comprising:
a noncrystalline component selected from the group consisting of mono-, di-, or poly- acrylates and methacrylates, unsaturated amides, vinyl compounds, and combinations thereof;
a crystalline component selected from the group consisting of polyesters, polyethers, polyolefins, polythioethers, polyarylalkylenes, polysilanes, polyamides, polyurethanes, and polymeric materials having crystallizable pendant moieties and the following general formula: 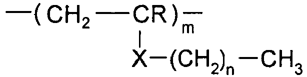 wherein:
R is hydrogen or a (C₁-C₄)alkyl group, X is --CH₂--, --C(O)O--, --O-C(O)--, -C(O)-NH--, --HN-C(O)--, --O--, --NH--, -O-C(O)-NH--, -HN-C(O)-O--, --HN-C(O)-NH--, or --Si(CH₃)₂--;
m is the number of repeating units in the polymer; and
n is great enough to provide sufficient side chain length and conformation to form polymers containing crystalline domains or regions, and combinations thereof;
greater than 60 wt-% of a filler system; and
an initiator system;
wherein the hardenable composition exhibits the sufficient malleability at a temperature of about 15°C to 38°C; with the proviso that if the filler system comprises fibers, the fibers are present in an amount of less than 20 wt-%, based on the total weight of the hardenable composition.

10. A dental article according to claim 1, wherein the hardenable composition comprises:
a resin system comprising a crystalline compound of the formula: 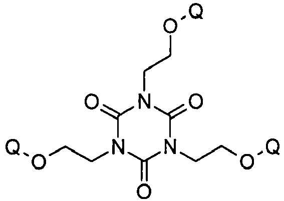 wherein each Q independently comprises polyester segments, polyamide segments, polyurethane segments, polyether segments, or combinations thereof;
a filler system; and
an initiator system.

11. A set of dental articles, the set comprising:
one or more preformed dental crowns (10; 20; 30) with a self-supporting side-generic external shape of an incisor, wherein the side-generic external shape is symmetrical about a plane of symmetry in facial-lingual direction (FL);
one or more preformed dental crowns (10; 20; 30) with a self-supporting side-generic external shape of a canine, wherein the side-generic external shape is symmetrical about a plane of symmetry in facial-lingual direction (FL);
one or more preformed dental crowns (10; 20; 30) with a self-supporting side-generic external shape of a premolar, wherein the side-generic external shape is symmetrical about a plane of symmetry in the facial-lingual direction (FL);
one or more preformed dental crowns (10; 20; 30) with a self-supporting side-generic external shape of a molar, wherein the side-generic external shape is symmetrical about a plane of symmetry in the facial-lingual direction (FL); and
a package containing the set of dental articles;
wherein each preformed dental crown (10; 20; 30) in the set of preformed dental crowns comprises a hardenable composition that has sufficient malleability such that the side-generic external shape can be formed into a left-side specific or right-side specific adjusted external shape.

12. A set according to claim 11, wherein the one or more preformed dental crowns (10; 20; 30) with a self-supporting side-generic external shape of a premolar comprise a plane of symmetry in the mesial-distal direction (MD).

13. A set according to claim 11, wherein the one or more preformed dental crowns (10; 20; 30) with a self-supporting side-generic external shape of a molar comprise a plane of symmetry in the mesial-distal direction (MD).

14. A set according to claim 11, wherein the hardenable composition has sufficient malleability to be formed into the left-side specific or right-side specific adjusted external shape at a temperature of 15°C to 38°C.

15. A set according to claim 11, wherein the hardenable composition comprises a resin system comprising a crystalline component, greater than 60 wt-% of a filler system, and an initiator system, wherein the hardenable composition exhibits the sufficient malleability at a temperature of about 15°C to 38°C, with the proviso that if the filler system comprises fibers, the fibers are present in an amount of less than 20 wt-%, based on the total weight of the hardenable composition.

16. A set according to claim 11, wherein the hardenable composition comprises a resin system comprising a crystalline component, greater than 70 wt-% of a filler system, and an initiator system, with the proviso that if the filler system comprises fibers, the fibers are present in an amount of less than 20 wt-%, based on the total weight of the composition.

17. A set according to claim 11, wherein the hardenable composition comprises:
a resin system comprising at least one ethylenically unsaturated component and a crystalline component;
greater than 60 wt-% of a filler system; and
an initiator system;
wherein the hardenable composition exhibits the sufficient malleability at a temperature of about 15°C to 38°C.

18. A set according to claim 11, wherein the hardenable composition comprises:
a resin system comprising at least one ethylenically unsaturated component;
greater than 50 wt-% of a filler system at least a portion of which is an inorganic material comprising nanoscopic particles having an average primary particle size of no greater than about 50 nm;
an initiator system; and
a surfactant system.

19. A set according to claim 11, wherein the hardenable composition comprises:
a resin system comprising:
a noncrystalline component selected from the group consisting of mono-, di-, or poly- acrylates and methacrylates, unsaturated amides, vinyl compounds, and combinations thereof;
a crystalline component selected from the group consisting of polyesters, polyethers, polyolefins, polythioethers, polyarylalkylenes, polysilanes, polyamides, polyurethanes, and polymeric materials having crystallizable pendant moieties and the following general formula: 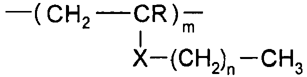 wherein:
R is hydrogen or a (C₁-C₄)alkyl group, X is --CH₂--, --C(O)O--, --O-C(O)--, --C(O)-NH--, --HN-C(O)--, --O--, --NH--, -O-C(O)-NH-, -HN-C(O)-O-, --HN-C(O)-NH--, or --Si(CH₃)₂--;
m is the number of repeating units in the polymer; and
n is great enough to provide sufficient side chain length and conformation to form polymers containing crystalline domains or regions, and combinations thereof;
greater than 60 wt-% of a filler system; and
an initiator system;
wherein the hardenable composition exhibits the sufficient malleability at a temperature of about 15°C to 38°C; with the proviso that if the filler system comprises fibers, the fibers are present in an amount of less than 20 wt-%, based on the total weight of the hardenable composition.

20. A set according to claim 11, wherein the hardenable composition comprises:
a resin system comprising a crystalline compound of the formula: 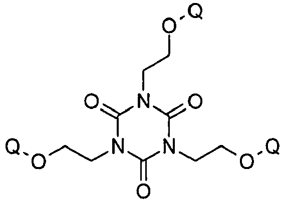 wherein each Q independently comprises polyester segments, polyamide segments, polyurethane segments, polyether segments, or combinations thereof;
a filler system; and
an initiator system.

## Patentansprüche

1. Dentalgegenstand, aufweisend:
eine vorgeformte Zahnkrone (10; 20; 30), die eine selbsttragende, seitenneutrale, äußere Form aufweist, ausgewählt aus der Gruppe bestehend aus Schneidezahn, Eckzahn, Prämolar und Molar;
wobei die seitenneutrale, äußere Form um eine Symmetrieebene in fazial-lingualer Richtung (FL) symmetrisch ist;
und wobei die vorgeformte Zahnkrone (10; 20; 30) eine härtbare Zusammensetzung aufweist, eine ausreichende Verformbarkeit hat, so dass die seitenneutrale, äußere Form in eine linksseitig spezifische oder rechtsseitig spezifische angepasste, äußere Form geformt werden kann.

2. Dentalgegenstand nach Anspruch 1, wobei die seitenneutrale, äußere Form ferner aus der Gruppe bestehend aus Prämolar und Molar ausgewählt wird; und wobei die seitenneutrale, äußere Form eine Symmetrieebene in mesial-distaler Richtung (MD) aufweist.

3. Dentalgegenstand nach Anspruch 1, wobei die vorgeformte Zahnkrone 20; 30; 60) einen inneren Hohlraum (62) aufweist, und wobei der innere Hohlraum (62) in lingualer oder fazialer Richtung versetzt ist.

4. Dentalgegenstand nach Anspruch 1, wobei die härtbare Zusammensetzung ausreichende Verformbarkeit hat, um bei einer Temperatur von 15 °C bis 38 °C in die linksseitig spezifische oder rechtsseitig spezifische angepasste, äußere Form geformt zu werden.

5. Dentalgegenstand nach Anspruch 1, wobei die härtbare Zusammensetzung ein Harzsystem aufweist, das einen kristallinen Bestandteil, mehr als 60 Gew.-% Füllmittelsystems und ein Initiatorsystem aufweist, wobei die härtbare Zusammensetzung die ausreichende Verformbarkeit bei einer Temperatur von ungefähr 15 °C bis 38 °C aufweist, mit der Maßgabe, dass, wenn das Füllmittelsystem Fasern aufweist, die Fasern in einem Anteil von weniger als 20 Gew.-% bezogen auf das Gesamtgewicht der härtbaren Zusammensetzung vorliegen.

6. Dentalgegenstand nach Anspruch 1, wobei die härtbare Zusammensetzung ein Harzsystem aufweist, das einen kristallinen Bestandteil, mehr als 70 Gew.-% eines Füllmittelsystems und ein Initiatorsystem aufweist, mit der Maßgabe, dass, wenn das Füllmittelsystem Fasern aufweist, die Fasern in einem Anteil von weniger als 20 Gew.-% bezogen auf das Gesamtgewicht der härtbaren Zusammensetzung vorliegen.

7. Dentalgegenstand nach Anspruch 1, wobei die härtbare Zusammensetzung Folgendes aufweist:
ein Harzsystem, das mindestens einen ethylenisch ungesättigten Bestandteil und einen kristallinen Bestandteil aufweist;
mehr als 60 Gew.-% eines Füllmittelsystems; und
ein Initiatorsystem;
wobei die härtbare Zusammensetzung die ausreichende Verformbarkeit bei einer Temperatur von ungefähr 15 °C bis 38 °C aufweist.

8. Dentalgegenstand nach Anspruch 1, wobei die härtbare Zusammensetzung aufweist:
ein Harzsystem, das mindestens einen ethylenisch ungesättigten Bestandteil aufweist;
mehr als 50 Gew.-% eines Füllmittelsystems mit mindestens einem Anteil anorganischen Materials, das nanoskopische Teilchen mit einer durchschnittlichen Primärteilchengröße von nicht mehr als ungefähr 50 nm aufweist;
ein Initiatorsystem; und
ein Tensidsystem.

9. Dentalgegenstand nach Anspruch 1, wobei die härtbare Zusammensetzung aufweist:
ein Harzsystem, aufweisend:
einen nichtkristallinen Bestandteil, ausgewählt aus der Gruppe bestehend aus Mono-, Di- oder Polyacrylaten und Methacrylaten, ungesättigten Amiden, Vinylverbindungen und Kombinationen daraus;
einen kristallinen Bestandteil, ausgewählt aus der Gruppe bestehend aus Polyestern, Polyethern, Polyolefinen, Polythioethern, Polyarylalkylenen, Polysilanen, Polyamiden, Polyurethanen und Polymermaterialien mit kristallisierbaren, hängenden Einheiten und der folgenden allgemeinen Formel: 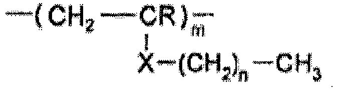 wobei:
R Wasserstoff oder eine (C₁-C₄)-Alkylgruppe und X --CH₂--, --C(O)O--, --O-C(O)--,--C(O)-NH--, --HN-C(O)--, --O--, --NH--, --O-C(O)-NH--,--HN-C(O)-O--, --HN-C(O)-NH--, oder --Si(CH₃)₂-- ist;
m die Anzahl der sich wiederholenden Einheiten in dem Polymer ist; und
n groß genug ist, um eine ausreichende Seitenkettenlänge und Konformation für die Bildung von Polymeren zu bieten, die kristalline Domänen oder Bereiche enthalten,
und Kombinationen daraus;
mehr als 60 Gew.-% eines Füllmittelsystems; und
ein Initiatorsystem;
wobei die härtbare Zusammensetzung die ausreichende Verformbarkeit bei einer Temperatur von ungefähr 15 °C bis 38 °C aufweist; mit der Maßgabe, dass, wenn das Füllmittelsystem Fasern aufweist, die Fasern in einem Anteil von weniger als 20 Gew.-% bezogen auf das Gesamtgewicht der härtbaren Zusammensetzung vorliegen.

10. Dentalgegenstand nach Anspruch 1, wobei die härtbare Zusammensetzung aufweist:
ein Harzsystem, das eine kristalline Verbindung der Formel: 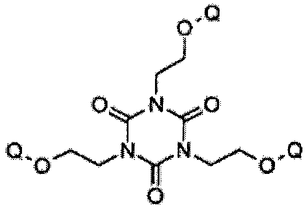 aufweist, wobei jedes Q unabhängig voneinander Polyestersegmente, Polyamidsegmente, Polyurethansegmente, Polyethersegmente oder Kombinationen daraus aufweist;
ein Füllmittelsystem; und
ein Initiatorsystem.

11. Satz von Dentalgegenständen, der aufweist:
eine oder mehrere vorgeformte Zahnkronen (10; 20; 30) mit einer selbsttragenden, seitenneutralen, äußeren Form eines Schneidezahns, wobei die seitenneutrale, äußere Form um eine Symmetrieebene in fazial-lingualer Richtung (FL) symmetrisch ist;
eine oder mehrere vorgeformte Zahnkronen (10; 20; 30) mit einer selbsttragenden, seitenneutralen, äußeren Form eines Eckzahns, wobei die seitenneutrale, äußere Form um eine Symmetrieebene in fazial-lingualer Richtung (FL) symmetrisch ist;
eine oder mehrere vorgeformte Zahnkronen (10; 20; 30) mit einer selbsttragenden, seitenneutralen, äußeren Form eines Prämolaren, wobei die seitenneutrale, äußere Form um eine Symmetrieebene in fazial-lingualer Richtung (FL) symmetrisch ist;
eine oder mehrere vorgeformte Zahnkronen (10; 20; 30) mit einer selbsttragenden, seitenneutralen, äußeren Form eines Molaren, wobei die seitenneutrale, äußere Form um eine Symmetrieebene in fazial-lingualer Richtung (FL) symmetrisch ist; und
eine Verpackung, die den Satz von Dentalgegenständen enthält;
wobei jede vorgeformte Zahnkrone (10; 20; 30) in dem Satz von vorgeformten Zahnkronen eine härtbare Zusammensetzung aufweist, die eine ausreichende Verformbarkeit hat, so dass die seitenneutrale, äußere Form in eine linksseitig spezifische oder rechtsseitig spezifische, angepasste, äußere Form geformt werden kann.

12. Satz nach Anspruch 11, wobei die eine oder die mehreren vorgeformten Zahnkronen (10; 20; 30) mit einer selbsttragenden, seitenneutralen, äußeren Form eines Prämolaren eine Symmetrieebene in mesial-distaler Richtung (MD) aufweist.

13. Satz nach Anspruch 11, wobei die eine oder die mehreren vorgeformten Zahnkronen (10; 20; 30) mit einer selbsttragenden, seitenneutralen, äußeren Form eines Molaren eine Symmetrieebene in mesial-distaler Richtung (MD) aufweist.

14. Satz nach Anspruch 11, wobei die härtbare Zusammensetzung eine ausreichende Verformbarkeit hat, um bei einer Temperatur von 15 °C bis 38 °C in eine linksseitig spezifische oder rechtsseitig spezifische, angepasste, äußere Form geformt zu werden.

15. Satz nach Anspruch 11, wobei die härtbare Zusammensetzung ein Harzsystem aufweist, das einen kristallinen Bestandteil, mehr als 60 Gew.-% eines Füllmittelsystems und ein Initiatorsystem aufweist, wobei die härtbare Zusammensetzung die ausreichende Verformbarkeit bei einer Temperatur von ungefähr 15 °C bis 38 °C aufweist, mit der Maßgabe, dass, wenn das Füllmittelsystem Fasern aufweist, die Fasern in einem Anteil von weniger als 20 Gew.-% bezogen auf das Gesamtgewicht der härtbaren Zusammensetzung vorliegen.

16. Satz nach Anspruch 11, wobei die härtbare Zusammensetzung ein Harzsystem aufweist, das einen kristallinen Bestandteil, mehr als 70 Gew.-% eines Füllmittelsystems und ein Initiatorsystem aufweist, mit der Maßgabe, dass, wenn das Füllmittelsystem Fasern aufweist, die Fasern in einem Anteil von weniger als 20 Gew.-% bezogen auf das Gesamtgewicht der härtbaren Zusammensetzung vorliegen.

17. Satz nach Anspruch 11, wobei die härtbare Zusammensetzung Folgendes aufweist:
ein Harzsystem, das mindestens einen ethylenisch ungesättigten Bestandteil und einen kristallinen Bestandteil aufweist;
mehr als 60 Gew.-% eines Füllmittelsystems; und
ein Initiatorsystem;
wobei die härtbare Zusammensetzung die ausreichende Verformbarkeit bei einer Temperatur von ungefähr 15 °C bis 38 °C aufweist.

18. Satz nach Anspruch 11, wobei die härtbare Zusammensetzung aufweist:
ein Harzsystem, das mindestens einen ethylenisch ungesättigten Bestandteil aufweist;
mehr als 50 Gew.-% eines Füllmittelsystems mit mindestens einem Anteil anorganischen Materials, das nanoskopische Teilchen mit einer durchschnittlichen Primärteilchengröße von nicht größer als ungefähr 50 nm aufweist;
ein Initiatorsystem; und
ein Tensidsystem.

19. Satz nach Anspruch 11, wobei die härtbare Zusammensetzung aufweist:
ein Harzsystem, aufweisend:
einen nichtkristallinen Bestandteil, ausgewählt aus der Gruppe bestehend aus Mono-, Di- oder Polyacrylaten und Methacrylaten, ungesättigten Amiden, Vinylverbindungen und Kombinationen daraus;
einen kristalline Bestandteil, ausgewählt aus der Gruppe bestehend aus Polyestern, Polyethern, Polyolefinen, Polythioethern, Polyarylalkylenen, Polysilanen, Polyamiden, Polyurethanen und Polymermaterialien mit kristallisierbaren, hängenden Einheiten und der folgenden allgemeinen Formel: 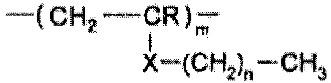 wobei:
R Wasserstoff oder eine (C₁-C₄)-Alkylgruppe und X --CH2--, --C(O)O--, --O-C(O)--,--C(O)-NH--, --HN-C(O)--, --O--, --NH--, --O-C(O)-NH--,--HN-C(O)-O--, --HN-C(O)-NH--, oder --Si(CH₃)₂-- ist;
m die Anzahl der sich wiederholenden Einheiten in dem Polymer ist; und
n groß genug ist, um eine ausreichende Seitenkettenlänge und Konformation für die Bildung von Polymeren zu bieten, die kristalline Domänen oder Bereicheanthalten,
und Kombinationen daraus;
mehr als 60 Gew.-% eines Füllmittelsystems; und
ein Initiatorsystem;
wobei die härtbare Zusammensetzung die ausreichende Verformbarkeit bei einer Temperatur von ungefähr 15 °C bis 38 °C aufweist; mit der Maßgabe, dass, wenn das Füllmittelsystem Fasern aufweist, die Fasern in einem Anteil von weniger als 20 Gew.-% bezogen auf das Gesamtgewicht der härtbaren Zusammensetzung vorliegen.

20. Satz nach Anspruch 11, wobei die härtbare Zusammensetzung aufweist:
ein Harzsystem, das eine kristalline Verbindung der Formel 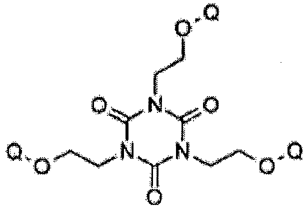 aufweist, wobei jedes Q unabhängig voneinander Polyestersegmente, Polyamidsegmente, Polyurethansegmente, Polyethersegmente oder Kombinationen daraus aufweist;
ein Füllmittelsystem; und
ein Initiatorsystem.

## Revendications

1. Article dentaire comprenant :
une couronne dentaire préformée (10 ; 20 ; 30) qui comprend une forme externe autoporteuse à latéralité générique choisie dans le groupe constitué d'incisive, canine, prémolaire et molaire ;
dans lequel la forme externe à latéralité générique est symétrique autour d'un plan de symétrie dans la direction faciale-linguale (FL) ;
et dans lequel la couronne dentaire préformée (10 ; 20 ; 30) comprend une composition durcissable qui a une malléabilité suffisante de sorte que la forme externe à latéralité générique peut être formée en une forme externe ajustée spécifique pour le côté gauche ou spécifique pour le côté droit.

2. Article dentaire selon la revendication 1, dans lequel la forme externe à latéralité générique est en outre choisie dans le groupe constitué de prémolaire et molaire ; et dans lequel la forme externe à latéralité générique comprend un plan de symétrie dans la direction mésiale-distale (MD).

3. Article dentaire selon la revendication 1, dans lequel la couronne dentaire préformée (10 ; 20 ; 30 ; 60) comprend une cavité interne (62) et dans lequel la cavité interne (62) est décalée vers la direction linguale ou la direction faciale.

4. Article dentaire selon la revendication 1, dans lequel la composition durcissable a une malléabilité suffisante pour être formée en une forme externe ajustée spécifique pour le côté gauche ou spécifique pour le côté droit à une température de 15 °C à 38 °C.

5. Article dentaire selon la revendication 1, dans lequel la composition durcissable comprend un système de résine comprenant un composant cristallin, plus de 60 % en poids d'un système de charge, et un système d'inducteur, dans lequel la composition durcissable présente la malléabilité suffisante à une température d'environ 15 °C à 38 °C, à condition que si le système de charge comprend des fibres, les fibres soient présentes en une quantité inférieure à 20 % en poids, sur la base du poids total de la composition durcissable.

6. Article dentaire selon la revendication 1, dans lequel la composition durcissable comprend un système de résine comprenant un composant cristallin, plus de 70 % en poids d'un système de charge, et un système d'inducteur, à condition que si le système de charge comprend des fibres, les fibres soient présentes en une quantité inférieure à 20 % en poids, sur la base du poids total de la composition.

7. Article dentaire selon la revendication 1, dans lequel la composition durcissable comprend :
un système de résine comprenant au moins un composant à insaturation éthylénique et un composant cristallin ;
plus de 60 % en poids d'un système de charge ; et
un système d'inducteur ;
dans lequel la composition durcissable présente la malléabilité suffisante à une température d'environ 15 °C à 38 °C.

8. Article dentaire selon la revendication 1, dans lequel la composition durcissable comprend :
un système de résine comprenant au moins un composant à insaturation éthylénique ;
plus de 50 % en poids d'un système de charge dont au moins une partie est un matériau inorganique comprenant des particules nanoscopiques possédant une taille moyenne de particules primaires n'excédant pas environ 50 nm ;
un système d'inducteur ; et
un système tensioactif.

9. Article dentaire selon la revendication 1, dans lequel la composition durcissable comprend :
un système de résine comprenant :
un composant non cristallin choisi parmi le groupe constitué de mono-, di- ou poly- acrylates et méthacrylates, amides insaturés, composés vinyliques, et des combinaisons de ceux-ci ;
un composant cristallin choisi parmi le groupe constitué de polyesters, polyéthers, polyoléfines, polythioéthers, polyarylalkylènes, polysilanes, polyamides, polyuréthanes et matériaux polymères ayant des fragments greffés cristallisables et la formule générale suivante : 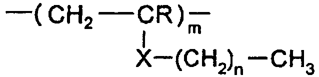 dans laquelle :
R est un hydrogène ou un groupe alkyle en (C₁ à C₄), X est --CH₂--, --C(O)O--, --O-C(O)--,--C(O)-NH--, --HN-C(O)--, --O--, --NH--, --O-C(O)-NH--,--HN-C(O)-O--, --HN-C(O)-NH--, ou --Si(CH₃)₂-- ;
m est le nombre de motifs répétés dans le polymère ; et
n est suffisamment grand pour fournir une longueur de chaîne latérale et une adaptation suffisantes pour former des polymères contenant des régions ou domaines cristallins,
et des combinaisons de ceux-ci ;
plus de 60 % en poids d'un système de charge ; et
un système d'inducteur ;
dans lequel la composition durcissable présente la malléabilité suffisante à une température d'environ 15 °C à 38 °C ; à condition que si le système de charge comprend des fibres, les fibres soient présentes en une quantité inférieure à 20 % en poids, sur la base du poids total de la composition durcissable.

10. Article dentaire selon la revendication 1, dans lequel la composition durcissable comprend :
un système de résine comprenant un composé cristallin de formule : 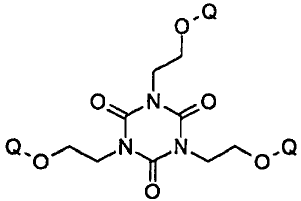 dans laquelle chaque Q comprend indépendamment des segments polyester, des segments polyamide, des segments polyuréthane, des segments polyéther, ou des combinaisons de ceux-ci ;
un système de charge ; et
un système d'inducteur.

11. Ensemble d'articles dentaires, l'ensemble comprenant :
une ou plusieurs couronnes dentaires préformées (10 ; 20 ; 30) avec une forme externe autoporteuse à latéralité générique d'une incisive, dans lequel la forme externe à latéralité générique est symétrique autour d'un plan de symétrie dans la direction faciale-linguale (FL) ;
une ou plusieurs couronnes dentaires préformées (10 ; 20 ; 30) avec une forme externe autoporteuse à latéralité générique d'une canine, dans lequel la forme externe à latéralité générique est symétrique autour d'un plan de symétrie dans la direction faciale-linguale (FL) ;
une ou plusieurs couronnes dentaires préformées (10 ; 20 ; 30) avec une forme externe autoporteuse à latéralité générique d'une prémolaire, dans lequel la forme externe à latéralité générique est symétrique autour d'un plan de symétrie dans la direction faciale-linguale (FL) ;
une ou plusieurs couronnes dentaires préformées (10 ; 20 ; 30) avec une forme externe autoporteuse à latéralité générique d'une molaire, dans lequel la forme externe à latéralité générique est symétrique autour d'un plan de symétrie dans la direction faciale-linguale (FL) ; et
un emballage contenant l'ensemble d'articles dentaires ;
dans lequel chaque couronne dentaire préformée (10 ; 20 ; 30) dans l'ensemble de couronnes dentaires préformées comprend une composition durcissable qui a une malléabilité suffisante de telle sorte que la forme externe à latéralité générique peut être formée en une forme externe ajustée spécifique pour le côté gauche ou spécifique pour le côté droit.

12. Ensemble selon la revendication 11, dans lequel la ou les couronnes dentaires préformées (10 ; 20 ; 30) avec une forme externe autoporteuse à latéralité générique d'une prémolaire comprennent un plan de symétrie dans la direction mésiale-distale (MD).

13. Ensemble selon la revendication 11, dans lequel la ou les couronnes dentaires préformées (10 ; 20 ; 30) avec une forme externe autoporteuse à latéralité générique d'une molaire comprennent un plan de symétrie dans la direction mésiale-distale (MD).

14. Ensemble selon la revendication 11, dans lequel la composition durcissable a une malléabilité suffisante pour être formée en une forme externe ajustée spécifique pour le côté gauche ou spécifique pour le côté droit à une température de 15 °C à 38 °C.

15. Ensemble selon la revendication 11, dans lequel la composition durcissable comprend un système de résine comprenant un composant cristallin, plus de 60 % en poids d'un système de charge, et un système d'inducteur, dans lequel la composition durcissable présente la malléabilité suffisante à une température d'environ 15 °C à 38 °C, à condition que si le système de charge comprend des fibres, les fibres soient présentes en une quantité inférieure à 20 % en poids, sur la base du poids total de la composition durcissable.

16. Ensemble selon la revendication 11, dans lequel la composition durcissable comprend un système de résine comprenant un composant cristallin, plus de 70 % en poids d'un système de charge, et un système d'inducteur, à condition que si le système de charge comprend des fibres, les fibres soient présentes en une quantité inférieure à 20 % en poids, sur la base du poids total de la composition.

17. Ensemble selon la revendication 11, dans lequel la composition durcissable comprend :
un système de résine comprenant au moins un composant à insaturation éthylénique et un composant cristallin ;
plus de 60 % en poids d'un système de charge ; et
un système d'inducteur ;
dans lequel la composition durcissable présente la malléabilité suffisante à une température d'environ 15 °C à 38 °C.

18. Ensemble selon la revendication 11, dans lequel la composition durcissable comprend :
un système de résine comprenant au moins un composant à insaturation éthylénique ;
plus de 50 % en poids d'un système de charge dont au moins une partie est un matériau inorganique comprenant des particules nanoscopiques possédant une taille moyenne de particules primaires n'excédant pas environ 50 nm ;
un système d'inducteur ; et
un système tensioactif.

19. Ensemble selon la revendication 11, dans lequel la composition durcissable comprend :
un système de résine comprenant :
un composant non cristallin choisi dans le groupe constitué de mono-, di-ou poly- acrylates et méthacrylates, amides insaturés, composés vinyliques, et des combinaisons de ceux-ci ;
un composant cristallin choisi parmi le groupe constitué de polyesters, polyéthers, polyoléfines, polythioéthers, polyarylalkylènes, polysilanes, polyamides, polyuréthanes et matériaux polymères comportant des fragments greffés cristallisables et la formule générale suivante : 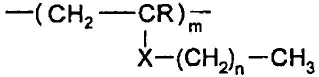 dans laquelle :
R est un hydrogène ou un groupe alkyle en (C₁ à C₄), X est --CH₂--, --C(O)O--, --O-C(O)--,--C(O)-NH--, --HN-C(O)--, --O--, --NH--, --O-C(O)-NH--, --HN-C(O)-O--, --HN-C(O)-NH--, ou --Si(CH₃)₂-- ;
m est le nombre de motifs répétés dans le polymère ; et
n est suffisamment grand pour fournir une longueur de chaîne latérale et une adaptation suffisantes pour former des polymères contenant des régions ou domaines cristallins,
et des combinaisons de ceux-ci ;
plus de 60 % en poids d'un système de charge ; et
un système d'inducteur ;
dans lequel la composition durcissable présente la malléabilité suffisante à une température d'environ 15 °C à 38 °C ; à condition que si le système de charge comprend des fibres, les fibres soient présentes en une quantité inférieure à 20 % en poids, sur la base du poids total de la composition durcissable.

20. Ensemble selon la revendication 11, dans lequel la composition durcissable comprend :
un système de résine comprenant un composé cristallin de formule : 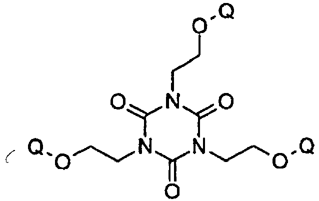 dans laquelle chaque Q comprend indépendamment des segments polyester, des segments polyamide, des segments polyuréthane, des segments polyéther, ou des combinaisons de ceux-ci ;
un système de charge ; et
un système d'inducteur.
